(19) Europäisches Patentamt
European Patent Office
Office européen des brevets

(11) Publication number: 0 430 484 A2

(12) **EUROPEAN PATENT APPLICATION**

(21) Application number: **90312391.7**

(22) Date of filing: **13.11.90**

(51) Int. Cl.5: **C07C 45/30**, C07C 49/175, C08G 65/32

(30) Priority: **01.12.89 US 444211**

(43) Date of publication of application: **05.06.91 Bulletin 91/23**

(84) Designated Contracting States: **DE FR GB Bulletin**

(71) Applicant: **TEXACO CHEMICAL COMPANY**
**3040 Post Oak Boulevard**
**Houston, Texas 77056(US)**

(72) Inventor: **Sanderson, John Ronald**
**P.O. Box 587**
**Leander, Texas 78641(US)**
Inventor: **Marquis, Edward Thomas**
**9004 Collinfield Drive**
**Austin, Texas 78758(US)**

(74) Representative: **Brock, Peter William et al**
**URQUHART-DYKES & LORD 91 Wimpole Street**
**London W1M 8AH(GB)**

(54) **Ketone derivatives of polyoxypropylene glycols.**

(57) A diketone having the formula:

(II)

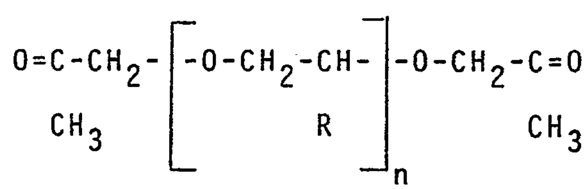

wherein R is hydrogen or methyl and n is 1 to 59, can be obtained by oxidizing a polyoxyalkylene glycol having an average molecular weight of 200 to 2,000 and having the formula:

(I)

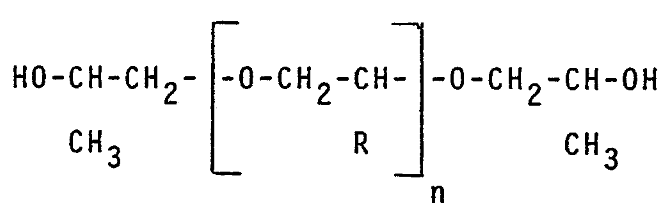

with an aqueous solution of an alkali metal or an alkaline earth metal hypochlorite in the presence of a $C_1$ - $C_4$halogenated alkane solvent and a ruthenium catalyst at a temperature of 10 to 50° C and a pressure of 0.1 to 7 MPa (0 to 1,000 psig) over a period of 10 to 20 hours.

EP 0 430 484 A2

# KETONE DERIVATIVES OF POLYOXYPROPYLENE GLYCOLS

This invention relates to the preparation of ketone derivatives of polyoxypropylene glycols. More particularly, this invention relates to a method wherein the terminal hydroxyl groups of a polyoxypropylene glycol are oxidized to ketone groups. Still more particularly, this invention is directed to a method wherein a polyoxypropylene glycol is brought into contact with a hypochlorite oxidant in the presence of a catalytically effective amount of a ruthenium catalyst and a halogenated alkane solvent in order to convert the hydroxyl groups of the polyoxypropylene glycol substantially selectively into terminal ketone groups. The ketone terminated derivatives of polyoxypropylene glycols are useful as intermediates for the preparation of a wide variety of products. For example, they may be reacted with amines to provide fuel additives or converted into carboxylic acids to provide surfactants.

It is known to oxidize secondary alcohols and primary benzyl and allyl alcohols to the corresponding ketones and aldehydes, in the presence of an oxidant, such as $Cu(NO_3)_2$or $Zn(NO_3)_2$supported on silica gel, in the presence of an aliphatic hydrocarbon solvent or a chlorinated aliphatic hydrocarbon solvent as shown, for example, by Takeshi Nishiguchi and Fumi Asano (J. Org. Chem. 1989, 54, 1531-1535).

US-A-4233460 discloses a process for converting alkoxyalkanols into the corresponding acids by reacting the alcohol with an alkali metal hydroxide and tertiary butyl hydroperoxide in the presence of a catalytic amount of palladium. The oxidation of polyethylene glycols to dicarboxylic acids by oxidation is an aqueous solution over a fixed bed of a catalyst consisting of platinum on a granular carbon support is disclosed in US-A-4256916.

US-A-4488944 discloses the preparation of dicarboxylic acids by the oxidation of polyalkylene glycols with electrochemically generated nickel-oxide hydroxide.

US-A-3479403 discloses that ruthenium can be used as an oxidation catalyst, and that activity is enhanced by maintaining the oxidation potential of the ruthenium catalyst between that of Ru(VIII) and that of Ru(IV). Example I discloses the oxidation of ethanol to acetic acid by the slow addition of an aqueous solution of calcium hypochlorite to an aqueous solution of ethanol containing a ruthenium chloride catalyst, the ruthenium chloride being oxidized to ruthenium tetraoxide. The oxidation of isopropanol to acetone with sodium hypochlorite in the presence of a ruthenium trichloride catalyst is also disclosed in Table II of US-A-3479403.

Barak et al . (J. Org. Chem., 1988, Vol . 53, pp. 3553-3555) disclose that secondary alcohols can be oxidized to ketones with one hundred percent selectivity when using an $H_2O_2$- $RuCl_3$catalyst system under phase-transfer conditions. Wolfe et al. (Chemical Communications, 1970, pp. 1420-1421) disclose that in the catalytic hypochlorite oxidation of organic compounds with ruthenium trichloride, the ruthenium trichloride is oxidized to ruthenium tetraoxide.

Anelli et al. (J. Org. Chem., 1987, Vol. 52, pp. 2559-2562) disclose oxidation of a variety of alcohols in solution in methylene chloride with sodium hypochlorite.

In all of these references (and in references not cited here) oxidation of polyalkylene glycols has always been on polyethylene glycols. As far as we are aware, there have been no references on the oxidation of a polypropylene glycol to diketones. This is especially surprising in view of the fact that lower molecular weight secondary alcohols have been oxidized to ketones.

In accordance with the present invention, a polyoxyalkylene glycol having a molecular weight of 200 to 2,000 and having the formula:

$$(I)\qquad HO\text{-}\underset{CH_3}{CH}\text{-}CH_2\text{-}\left[\text{-}O\text{-}CH_2\text{-}\underset{R}{CH}\text{-}\right]_n\text{-}O\text{-}CH_2\text{-}\underset{CH_3}{CH}\text{-}OH$$

wherein R is hydrogen or methyl, and n is 1 to 59, can be oxidized in the presence of a halogenated alkane solvent and a ruthenium catalyst with an alkali metal or alkaline earth metal hypochlorite at a temperature of 10 to 50 °C and a pressure of 0.1 to 7 MPa (0 to 1,000 psig) over a period of 10 to 20 hours to provide the corresponding diketone having the formula:

(II)

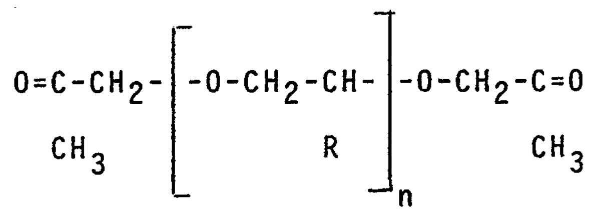

wherein R and n have the meaning given above.

The thus-prepared diketones are useful, for example, as intermediates for conversion into carboxylic acids to provide surfactants, and for reaction with amine adducts to provide fuel additives.

In accordance with the preferred embodiment of the present invention, a polyoxypropylene glycol having a molecular weight of 200 to 1,000 is added to a reaction zone together with 4 to 20 moles, per mole of polyoxypropylene glycol , of a halogenated methane solvent such as methylene chloride, and a catalytically effective amount of a ruthenium oxide catalyst. Thereafter, over a period of 10 to 20 hours, from 2 to 4 moles of an alkali metal hypochlorite, per mole of polyoxypropylene polyol, is added at a temperature of 10 to 50 °C and a pressure of 0.1 to 3.5 MPa (0 to 500 psig) to convert the polyoxypropylene glycol substantially quantitatively into the corresponding diketone. Still more preferably, the oxidation reaction is conducted in the presence of 0.5 to 5 moles of an alkali metal bicarbonate per mole of hypochlorite oxidant.

The starting materials for the present invention include a polyoxyalkylene glycol, as hereinafter defined, a $C_1$ -$C_4$halogenated alkane solvent, a ruthenium catalyst, an alkali metal or alkaline earth metal hypochlorite and, optionally, an alkali metal bicarbonate.

The ruthenium-containing compounds employed as catalysts may take different forms. For instance, the ruthenium may be added to the reaction mixture in an oxide form, as in the case of, for example, ruthenium (IV) oxide hydrate, anhydrous ruthenium (IV) dioxide and ruthenium (VIII) tetraoxide. Alternatively, it may be added as the salt of a mineral acid, as in the case of ruthenium (III) chloride hydrate, ruthenium (III) bromide, ruthenium (III) iodide or tricarbonylruthenium nitrate; or as the salt of a suitable organic carboxylic acid, for example, ruthenium (III) acetate, ruthenium naphthenate or ruthenium valerate; or as ruthenium complexes with carbonyl-containing ligands such as ruthenium (III) acetylacetonate. The ruthenium may also be added to the reaction zone as a carbonyl or hydrocarbonyl derivative. Suitable examples include, among others, triruthenium dodecacarbonyl and other hydrocarbonyls such as the tricarbonylruthenium (II) chloride dimer $(Ru(C0)_3Cl_2)_2$.

Preferred ruthenium-containing compounds include oxides of ruthenium, ruthenium salts of an organic carbonylic acid and ruthenium carbonyl or hydrocarbonyl derivatives. Among these are ruthenium (IV) dioxide hydrate, ruthenium (VIII) tetraoxide, anhydrous ruthenium (IV) oxide, ruthenium acetate, ruthenium propionate, ruthenium (III) acetylacetonate, and triruthenium dodecacarbonyl.

Additional examples of ruthenium compounds include ruthenium octoate, ruthenium laurate, ruthenium stearate, ruthenium linoleate, ruthenium nitrate, ruthenium sulfate and ruthenium carbonyl.

The polyoxyalkylene glycol feedstock to be used in accordance with the present invention is a polyoxyalkylene glycol having an average molecular weight of 200 to 2,000 and having the formula:

(I)

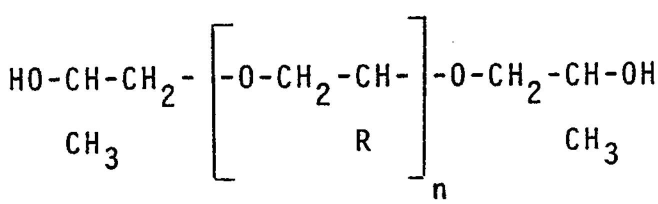

wherein R is hydrogen or methyl and n is 1 to 59.

A preferred group of polyoxyalkylene glycols to be used as feedstocks are polyoxypropylene glycols having the formula:

(III) 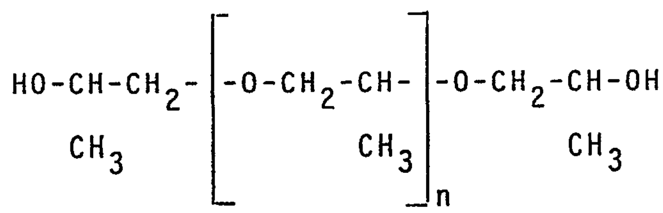

wherein n is 1 to 40.

Representative products of this nature include, for example, polyoxypropylene glycols having average molecular weights of about 230, of about 400, and of about 2,000, all of which are sold by the Texaco Chemical Company.

Another group of polyoxyalkylene glycols that can be used to practice the present invention are compounds having the formula:

(IV) 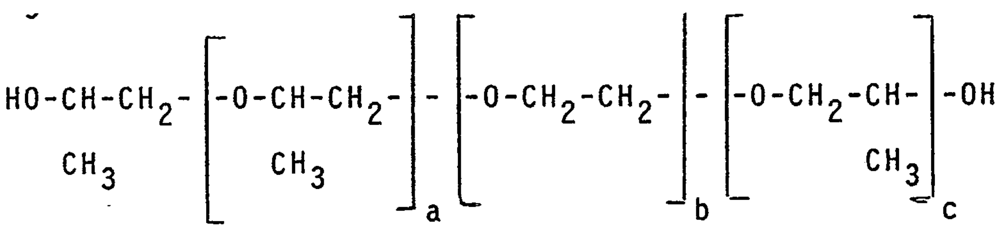

wherein a+c is 2 to 10, and b is 1 to 50.

Representative products include a polyoxyethylene/ oxypropylene glycol wherein a+c is 2.5 and b is 8.5, and the product has a molecular weight of about 600, a poly(oxyethylene/oxypropylene) glycol having an average molecular weight of about 900 wherein a+c is about 2.5 and b is about 15.5, and a poly-(oxypropylene/oxyethylene) glycol having an average molecular weight of about 2,000 wherein a+c is about 2.5 and b is about 40.5

The solvent is a halogenated $C_1$ -$C_4$alkane solvent, preferably a halogenated methane such as methylene chloride. However, other solvents may be used such as 1,1,1-trichloroethane or chloroflorocarbons.

The oxidant to be used in accordance with the present invention is an alkali metal or alkaline earth metal hypochlorite such as sodium hypochlorite, calcium hypochlorite, or potassium hypochlorite.

The hypochlorite oxidant is preferably employed in the form of 5 to 25 wt.% aqueous solution, in an amount of 2 to 4 moles of hypochlorite per mole of polyol.

In the reaction procedure to be used in practicing the process of the present invention, the polyoxyalkylene glycol, the solvent and the ruthenium catalyst are added to a suitable reaction vessel, such as an autoclave, provided with appropriate agitation means and means for controlling temperature within the autoclave, such as a jacket through which a heat exchange fluid may be circulated.

From 4 to 20 moles of solvent, and, more preferably, from 5 to 10 moles, should be used per mole of polyoxyalkylene glycol.

The ruthenium catalyst should be added in a catalytically effective amount. The amount of catalyst to be used will be dependent upon the specific species of ruthenium starting material that is employed. In general, from 0.0005 to 0.1 moles, more preferably 0.0005 to 0.01 moles, of catalyst should be used per mole of hypochlorite oxidant.

The hypochlorite oxidant is preferably employed in the form of 5 to 25 wt.% aqueous solution of the hypochlorite.

In accordance with a preferred embodiment of the present invention, from 0.5 to 5 moles of an alkali metal bicarbonate, preferably sodium bicarbonate, is employed per mole of hypochlorite oxidant, in order to enhance selectivity.

The reaction is conducted at a temperature of 10 to 50° C, and at a pressure of 0.1 to 7 MPa (0 to 1000 psig) and more preferably 0.1 to 3.5 MPa (0 to 500 psig) for a period of 10 to 20 hours, more preferably 10 to 15 hours.

As a result, the polyoxyalkylene glycol feedstock will be substantially selectively converted into the corresponding diketone derivative having the formula:

(II) 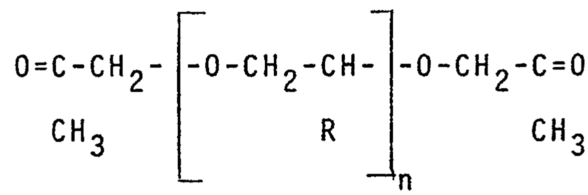

wherein R is hydrogen or methyl and n is 1 to 50.

## EXAMPLES

The invention will be further illustrated by the following specific examples.

### Procedure:

Polypropylene glycol having a molecular weight of 230 (in an amount of 10g), solvent, and catalyst(s) were charged to a 3-necked flask equipped with stirrer, water bath (or heating mantle), thermometer, water-cooled condenser and addition funnel. The oxidant was added slowly to the stirred reaction mixture over several hours. The reaction mixture was then stirred for an additional 15 hours. The aqueous layer was separated from the organic layer and 50g of concentrated HCl added to the aqueous layer. The aqueous layer was saturated with NaCl , and extracted with 3 x 50 ml of $CH_2Cl_2$. The $CH_2Cl_2$fractions were all combined and dried over anhydrous sodium sulphate. The methylene chloride was then removed on a rotary evaporator and the residue was analyzed by IR. Some runs were confirmed by NMR analysis. The results are shown in the attached Table I.
(TBHP is t-butyl hydroperoxide.)

TABLE I

| Run Number | Catalyst[b] | (g) | Oxidant | (g) | Solvent | (g) | Time (Hr) | Temp (°C) | % Ketone to Alcohol | Remarks |
|---|---|---|---|---|---|---|---|---|---|---|
| 1 | $RuO_2$ | 0.020 | 5% NaOCl | 300 | $CH_2Cl_2$ | 90 | 15 | 38 | 91 | Gentle reflux |
| 2 | $RuO_2$ | 0.010 | 5% NaOCl | 300 | $CH_2Cl_2$ | 90 | 15 | 25 | 49 | $H_2O$ bath |
| 3 | $RuO_2$ | 0.020 | 5% NaOCl | 300 | $CH_2Cl_2$ | 90 | 15 | 25 | 76 | $H_2O$ bath |
| 4 | $RuO_2$ | 0.010 | 5% NaOCl | 300 | None | - | 15 | 25 | 26 | $H_2O$ bath |
| 5 | $RuO_2$ | 0.020 | 5% NaOCl | 300 | None | - | 15 | 25 | 34 | $H_2O$ bath |
| 6 | $RuO_2$ | 0.010 | 90% TBHP | 20 | $CH_2Cl_2$ | 90 | 15 | 38 | 19 | Gentle reflux |
| 7 | $RuO_2$ | 0.020 | 90% TBHP | 20 | $CH_2Cl_2$ | 90 | 15 | 38 | 22 | Gentle reflux |
| 8 | None | - | 10% NaOCl | 150 | $CH_2Cl_2$ | 90 | 15 | 38 | 8 | Gentle reflux |
| 9 | $RuO_2$ | 0.020 | 10% NaOCl | 300 | $CH_2Cl_2$ | 90 | 15 | 25 | 67 | $H_2O$ bath |
| 10 | $RuO_2$ | 0.020 | 10% NaOCl | 300 | $CH_2Cl_2$ | 90 | 16 | 38 | 30 | Gentle reflux |
| 11 | $RuO_2$ | 0.10 | 10% NaOCl | 300 | $CH_2Cl_2$ | 90 | 15 | 38 | 92 | Gentle reflux |
| 12 | $RuO_2$ | 0.040 | 10% NaOCl | 300 | $CH_2Cl_2$ | 90 | 15 | 38 | 84 | Gentle reflux |
| 13 | $Co_3O_4$ | 1.0 | 10% NaOCl | 300 | $CH_2Cl_2$ | 90 | 15 | 38 | 11 | Gentle reflux |
| 14 | CuO | 1.0 | 10% NaOCl | 150 | $CH_2Cl_2$ | 90 | 15 | 38 | 11 | Gentle reflux |
| 15 | $Fe_3O_4$ | 1.0 | 10% NaOCl | 300 | $CH_2Cl_2$ | 90 | 15 | 38 | 9 | Gentle reflux |
| 16 | $Mn_3O_4$ | 1.0 | 10% NaOCl | 300 | $CH_2Cl_2$ | 90 | 15 | 38 | 10 | Gentle reflux |

As will be noted from the results set forth in Table I, the polyoxypropylene feedstock was substantially completely converted into the corresponding diketone in runs 1 and 11 which are conducted in accordance with the present invention.

However, when the temperature and the amount of ruthenium catalyst were both reduced in run 2, the conversion into the diketone was only about 50%. Use of an increased amount of catalyst, but a lower temperature of 25° C, only resulted in about a 75% conversion into the diketone. In the absence of a solvent (runs 4 and 5), the conversions were very poor, ranging from 25 to 35%. Use of tertiary butyl hydroperoxide rather than sodium hypochlorite in runs 6 and 7 also led to adverse results. Oxidation of the polyoxypropylene glycol in the absence of the ruthenium catalyst in run 8 resulted in only about 8% conversion into the diketone.

In run 9, when the reaction temperature was only 25° C, there was again a poor conversion to the diketone.

In run 10, where the reaction time was 6 hours, the conversion into the diketone was only 30%, whereas in run 11, with a reaotion time of 15 hours, the conversion into the diketone was 92%.

The remaining runs in Table I wherein different catalysts such as cobalt, copper oxide, iron and manganese were used also gave adverse results.

In accordance with the preferred embodiment of the present invention, an alkali metal bicarbonate additive is used to enhance the selectivity to the diketone reaction product.

Example 2

Using the procedure of Example 1, a series of experiments were conducted using sodium bicarbonate as an additive. The proportions of the reactants used and the results obtained are summarized in Tables II, III and IV.

TABLE II

| Run Number | Catalyst | (g) | Additive | (g) | Oxidant | (g) | Solvent | (g) | Time (Hr) | Temp (°C) | % Ketone to Alcohol |
|---|---|---|---|---|---|---|---|---|---|---|---|
| 17 | $RuO_2$ | 0.020 | $NaHCO_3$ | 2.0 | 5% NaOCl | 300 | $CH_2Cl_2$ | 90 | 15 | 38 | 85 |
| 18 | $RuO_2$ | 0.020 | - | - | 5% NaOCl | 300 | $CH_2Cl_2$ | 90 | 15 | 38 | 91 |
| 19 | - | - | $NaHCO_3$ | 3.0 | 5% NaOCl | 300 | $CH_2Cl_2$ | 90 | 15 | 38 | 13 |
| 20 | - | - | $NaHCO_3$ | 3.0 | 5% NaOCl | 300 | $CH_2Cl_2$ | 90 | 15 | 25 | 18 |
| 21 | - | - | NaOH | 4.0 | 5% NaOCl | 300 | - | - | 15 | 25 | 14 |
| 22 | $RuO_2$ | 0.010 | NaOH | 4.0 | 5% NaOCl | 300 | $CH_2Cl_2$ | 90 | 15 | 25 | 5 |
| 23 | - | - | - | - | 10% NaOCl | 150 | $CH_2Cl_2$ | 90 | 15 | 38 | 2 |

TABLE III

RELATIVE MOL. % TERMINATION BY C-13 NMR

| Run No. | Ketone | Alcohol |
|---|---|---|
| 18 | 91 | 9 |
| 17 | 85 | 15 |

TABLE IV

PERCENTAGE OF TOTAL CARBON AREA BY C-13 NMR

| Run No. | Ketone | Other Carbonyl | Olefin |
|---|---|---|---|
| 18 | 5.4 | 1.2 | 0.6 |
| 17 | 4.6 | $<0.2$ | 0.3 |

The explanation of the results obtained in run 17 as compared with run 18 is as follows.

With respect to Table II, runs 17 and 18 are identical, except that $NaHCO_3$ was added in run 17. The conversion (as measured by IR) is actually slightly less than the run in the absence of $NaHCO_3$. At first glance, then, the presence of $NaHCO_3$ appears to be slightly detrimental. However, IR analysis measures only the conversion and not the selectivity.

Table III shows the relative mole % termination by C-13 NMR. Agreement with the results obtained by IR is excellent. Table IV shows the percentage of total carbon area by C-13 NMR. It is here that we see the superior results obtained in the presence of $NaHCO_3$. Note that olefin by-product has been cut in half and other carbonyl by-products have been reduced by more than a factor of 6!

Run 22, carried out in the presence of NaOH and $RuO_2$, showed only 5% conversion. $NaHCO_3$ alone (runs 19 and 20) and NaOH alone also showed poor results.

## Claims

**1.** A method for making a diketone having the formula:

(II) 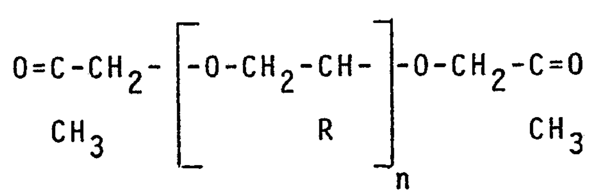

wherein R is hydrogen or methyl and n is 1 to 59 which comprises reacting a polyoxyalkylene glycol having an average molecular weight of 200 to 2,000 and having the formula:

(I) 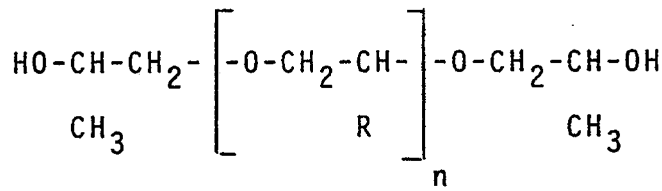

with an aqueous solution of an alkali metal or an alkaline earth metal hypochlorite oxidant in the presence of a $C_1$ -$C_4$ halogenated alkane solvent and a ruthenium catalyst with agitation at a temperature of 10 to 50 °C and a pressure of 0.1 to 7 MPa (0 to 1,000 psig) over a period of 10 to 20 hours.

**2.** A method according to Claim 1 characterized in that the polyoxyalkylene glycol is a polyoxypropylene diol having the formula:

(III) 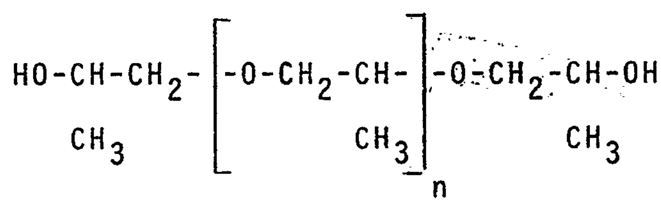

wherein n is 1 to 40.

**3.** A method according to Claim 1 characterized in that the polyoxyalkylene glycol is a diol having the formula:

(IV) 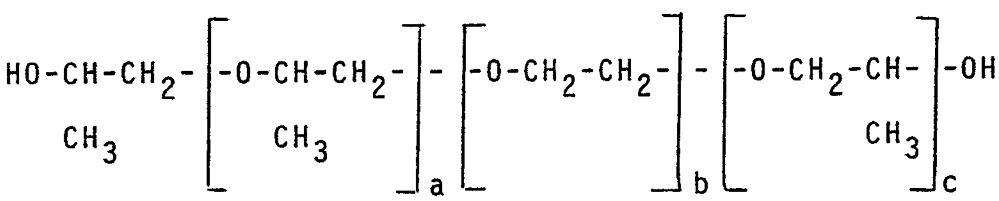

wherein a + c equals 2 to 10 and b is 1 to 50.

**4.** A method according to any one of Claims 1 to 4 characterized in that the reaction is carried out in the presence of an alkali metal bicarbonate.

**5.** A method according to Claim 4 wherein the alkali metal bicarbonate is used in an amount from 0.5 to 5 mols per mole of hypochlorite.

**6.** A method according to any one of Claims 1 to 5 characterized in that the halogenated alkane solvent comprises from 4 to 20 mols of halogenated methane per mole of polyoxyalkylene glycol.

**7.** A method according to any one of Claims 1 to 6 characterized in that the ruthenium catalyst is a ruthenium oxide.

**8.** A method according to any one of Claims 1 to 7 characterized in that the hypochlorite is sodium hypochlorite.

**9.** A method according to any one of Claims 1 to 8 characterized in that the halogenated alkane is methylene chloride.

**10.** A method according to any one of Claims 1 to 9 characterized in that the aqueous solution of the hypochlorite is added to the halogenated alkane, the ruthenium catalyst and the polyoxyalkylene glycol

over a period of 10 to 20 hours.

**11.** A method according to any one of Claims 1 to 10 characterized in that the ruthenium catalyst is used in an amount of 0.0005 to 0.1 moles per mole of hypochlorite.